(19) Europäisches Patentamt
European Patent Office
Office européen des brevets

(11) Numéro de publication : **0 554 343 B1**

(12) **FASCICULE DE BREVET EUROPEEN**

(45) Date de publication du fascicule du brevet :
**29.06.94 Bulletin 94/26**

(51) Int. Cl.$^5$ : **A61K 7/00,** A61K 7/48,
A61K 9/127, A61K 35/08,
A61K 47/34

(21) Numéro de dépôt : **91919483.7**

(22) Date de dépôt : **16.10.91**

(86) Numéro de dépôt international :
**PCT/FR91/00805**

(87) Numéro de publication internationale :
**WO 92/06666 30.04.92 Gazette 92/10**

(54) **LIPOSOMES D'EAUX THERMALES STABILISES DANS UN GEL D'ADN.**

(30) Priorité : **17.10.90 FR 9012811**

(43) Date de publication de la demande :
**11.08.93 Bulletin 93/32**

(45) Mention de la délivrance du brevet :
**29.06.94 Bulletin 94/26**

(84) Etats contractants désignés :
**AT BE CH DE DK ES FR GB GR IT LI LU NL SE**

(56) Documents cités :
**EP-A- 0 274 363
EP-A- 0 349 429
FR-A- 2 511 243
FR-A- 2 609 393
FR-A- 2 622 104**

(73) Titulaire : **PIERRE FABRE COSMETIQUE
45, Place Abel Gance
F-92100 Boulogne (FR)**

(72) Inventeur : **FABRE, Pierre
1, avenue d'Albi
F-81100 Castres (FR)**
Inventeur : **COUSSE, Henri
La Foun de las Nobios,
Chemin de Lastinos
F-81100 Castres (FR)**
Inventeur : **MOUZIN, Gilbert
54, rue d'Austerlitz
F-81100 Castres (FR)**
Inventeur : **TREBOSC, Marie-Thérèse
19, rue Baron-Cachin
F-81100 Castres (FR)**

(74) Mandataire : **Warcoin, Jacques et al
Cabinet Régimbeau
26, avenue Kléber
F-75116 Paris (FR)**

EP 0 554 343 B1

## Description

La présente invention concerne des compositions à base d'eau thermale comportant des liposomes d'eau thermale stabilisés dans un gel d'ADN, utiles notamment en dermatologie et en cosmétologie.

Les liposomes sont des micro-vésicules constitués d'une ou plusieurs bicouches lipidiques délimitant un espace aqueux central et en cas de liposomes multi-lamellaires, un comportement aqueux entre deux bicouches.

Ces structures sont composées de phopholipides auxquels on ajoute fréquemment des stérols tels que le cholestérol pour en augmenter la stabilité.

Les liposomes peuvent être classés selon leur taille et leur caractère uni ou multi-lamellaire.

MLV (Multi-Lamellar Vesicles)       : diamètre 100 à 5000 nm (plusieurs bicouches)
LUV (Large Unilamellar Vesicles)     : diamètre 200 à 2000 nm (1 bicouche)
SUV (Small Unilamellar Vesicles)     : diamètre 20 à 80 nm (1 bicouche)

Des techniques permettant d'obtenir de grandes quantités de liposomes ont été développées dans l'industrie (ultra-dispersion, sonication, lipopred.) Réf. PUISIEUX F., DELATTRE J. - Les liposomes. Application thérapeutiques. Technique et Documentation, Lavoisier Paris, 1985.

Ces micro-vésicules sont capables d'interéagir avec les cellules dont les membranes sont de nature identique à celle du liposome.

La dermatologie et la cosmétologie constituent des secteurs prometteurs pour l'exploitation des liposomes.

Les principales études concernant la peau sont relatives aux liposomes de corticoïdes. Il semblerait que la micro-encapsulation des corticoïdes diminue la pénétration percutanée du produit et augmente sa concentration au niveau des sites locaux : épiderme et derme. Réf. WOHLRAB W., LASCH J. - Penetration Kinetics of liposomal hydrocortisone in human skin. Dermatologica, 174, 18-22, 1987.

D'autres substances ont été incorporées mais les études sont trop parcellaires pour être généralisées : l'EGF (Epidermal Growth Factor) aiderait à la cicatrisation des plaies : les superoxydes dismutases auraient localement une action anti-inflammatoire. Malheureusement les vésicules de liposomes sont très fragiles dans les formulations utilisées et elles sont souvlent détruites avant d'atteindre leur cible.

De nombreux auteurs ont mentionné l'utilisation d'agents gélifiants pour stabiliser les vésicules liposomiales sous forme de gel aqueux.

Les principaux agents gélifiants utilisés sont : la gélatine, les polymères carboxyvinyliques, les polymères métacryliques, les copolymères de polydimethylsiloxane et plus récemment, le collagène.

La stabilisation des liposomes dans un gel aqueux avec les agents gélifiants actuellement utilisés ne permet pas d'obtenir une stabilité de la formule supérieure à 3 mois à la température de 40°C.

La présente invention permet de pallier à cet inconvénient majeur en stabilisant les liposomes dans un gel d'acide désoxyribonucléique,

Selon l'invention, l'ADN sera de l'ADN hautement polymérisé selon des procédés connus de l'homme de l'Art (ci-après "ADN HP") et disponible commercialement.

On peut citer en particulier de l'ADN présentant une masse moléculaire comprise entre 500.000 et 1 500 000 de préférence entre 800.000 et 1.200.000.

La composition selon l'invention contient avantageusement de 0,1 à 10 % en poids d'ADN et plus particulièrement de 0,5 à 5%.

Selon l'invention, on incorpore dans les liposomes des eaux thermales et plus particulièrement l'eau d'AVENE ou l'eau de CAUTERETS. L'eau d'AVENE présente des vertus thérapeutiques utiles dans le traitement des eczémas, prurits, psoriasis, retards de cicatrisation, brûlures. Des études fondamentales ont elles aussi apporté leur caution à l'efficacité de l'EAU D'AVENE. C'est ainsi que plusieurs séries d'études ont permis de démontrer que l'EAU D'AVENE exerce un effet inhibiteur sur la dégranulation des basophiles humaings. Elle inhibe également la migration des polynucléaires dont le rôle dans l'inflammation cutanée est important.

L'eau de CAUTERETS sulfurée présente, sous forme de liposomes, un intérêt en dermatologie et plus particulièrement dans les traitements suivants psoriasis, eczémas, acné, prurit, séborrhée, alopécie.

**La composition de l'EAU D'AVENE est la suivante :**

| ANION | mg/l |
|---|---|
| $HCO_3^-$ (bicarbonates)............................................. | 218,4 |
| $Cl^-$ (chlorures)................................................. | 5,8 |
| $SO_4^{--}$ (sulfates)................................................. | 12,4 |
| $NO_3$ (nitrates)................................................. | 1,1 |
| $NO_2$ (nitrites)................................................. | $<$ 0,02 |
| $F^-$ (fluorures................................................. | 0,12 |
| $PO_4^{---}$ (phosphates)............................................. | $<$ 0,1 |
| $Br^-$ (bromures)................................................. | $<$ 0,1 |
| **CATIONS** | |
| $Ca^{++}$ (calcium)................................................. | 40,8 |
| $Mg^{++}$ (magnésium)............................................. | 22,7 |
| $K^+$ (potassium)............................................. | 1,0 |
| $Na^+$ (sodium)................................................. | 4,8 |
| $Li^+$ (lithium)................................................. | $<$ 0,1 |
| $Fe^{++}$ (fer)..................................................... | $<$ 0.01 |
| $Mn^{++}$ (manganèse)............................................. | $<$ 0,005 |
| $Sr^{++}$ (strontium)............................................. | 0,13 |

L'eau de CAUTERETS a la composition suivante :

| ANIONS | | mg/l |
|---|---|---|
| $HCO^{--}_3$ (Bicarbonate).......................................................... | | 25 |
| $CO^{--}_3$ (Carbonates)......................................................... | | 23,4 |
| $H_3SiO^-_4$ (Silicates).............................................................. | | 32,8 |
| $Cl^-$ (Chlorures)........................................................ | | 45 |
| $SO^2_4{}^-$ (Sulfates).............................................................. | | 31,5 |
| $NO^-_2$ (Nitrites).............................................. | | — |
| $NO^-_3$ (Nitrates).............................................. | | — |
| $PO^3_4{}^-$ (Phosphates)...................................... | | — |
| $F^-$ (Fluorures)............................................ | | 2,2 |
| $HS^-$ (Sulfures)........................................... | | traces |
| $SO^2_3{}^-$ (Sulfites)............................................ | | traces |
| $S_2O^2_3{}^-$ (Triosulfates)..................................... | | 5,6 |
| **CATIONS** | | |
| $Ca^{2+}$ (Calcium)...................................... | | 5 |
| $Mg^{2+}$ (Magnésium...................................... | | 0,12 |
| $Na^+$ (Sodium).......................................... | | 63,6 |
| $K^+$ (Potassium)...................................... | | 1,8 |
| $NH^+_4$ (Ammonium)...................................... | | |
| $Mn^{2+}$ (Manganèse)...................................... | | |
| $Al^{3+}$ (Aluminium)...................................... | | |
| $Zn^{2+}$ (Zinc)........................................... | | |
| $Cu^{2+}$ (Cuivre).......................................... | | |
| $Li^+$ (Lithium)........................................ | | 0,18 |

Bien entendu, selon la présente invention, toutes autres eaux thermales présentant un intérêt en thérapeutique et/ou cosmétologique, peuvent également être micro-encapsulés afin de permettre une pénétration ciblée de ces eaux au niveau de l'épiderme et du derme;

Selon une caractéristique additionnelle de la présente invention, la composition contient éventuellement en outre, un principe actif associé tel que

. un agent antibactérien et plus particulièrement le phenonip, l'EDTA, l'acide benzoïque, le parahydroxybenzoate de butyle, l'acide sorbique.

. un principe vitaminique associé tel que la vitamine E, la vitamine C.

. ou encore une huile telle que de l'huile de bourrache ou de l'huile d'argan.

Bien entendu, la liste ci-dessus n'est pas limitative.

Ce principe actif associé sera alors présent en particulier dans une quantité 0 de 5 % et le plus souvent de 0,1 à 3 % en poids de la composition.

La composition selon la présente invention contient de façon avantageuse de 0,1 à 10 % en poids de lipides entrant dans la constitution des liposomes et plus particulièrement de 0,5 à 5 %, le rapport en poids (lipides)/(eau thermale encapsulée dans les liposomes) est environ de 1/3.

Dans un mode de réalisation, les liposomes utilisés dans la présente invention sont de type multilamellaires préparés selon le brevet français 2 634 375.

Dans ce mode de réalisation particulier, les gels de liposomes sont préparés de la manière suivante :

a) les liposomes

On prépare une phase liquide constituée essentiellement par une solution de lipides amphiphiles et éventuellement un dit principe actif associé de nature lypophile dans un solvant organique et plus particulièrement l'éthanol.

Cette phase est alors ajoutée sous agitation modérée, dans une solution d'eau thermale contenant éventuellement un dit principe associé de nature hydrophile.

Après évaporation sous pression réduite, on obtient une suspension de concentration voulue en liposomes.

Les lipides amphiphiles peuvent être des glycolipides, des phospho-aminolipides, et notamment les phospholipides, par exemple les lécithines (d'oeuf, de soja, etc...).

Le solvant est de préférence un alcool miscible à l'eau en toutes proportions, notamment l'éthanol.

La solution de lipides amphiphiles peut contenir en outre une substance de nature lipophile destinée à modifier les caractéristiques physiques (charge électrique, rigidité) ou chimique de la paroi telle que du cholestérol, de la stearylamine, de l'acide phosphatidique, etc...

La concentration des lipides dans le solvant peut être de 0,1 à 10 % en poids, de préférence 1 à 5 % en poids.

Il est avantageux que le volume de solvant utilisé pour la phase (1) soit compris entre 30 et 100 % ; par exemple environ 50 %, du volume d'eau de la phase (2), afin d'obtenir des liposomes de petite taille (notamment de 100 à 300 nm).

Ainsi l'invention permet d'obtenir des médicaments, notamment sous forme injectable et des produits cosmétiques qui sont très stables.

b) préparation du gel de liposomes :

A la suspension précédente, on introduit sous agitation modérée l'ADN (notamment l'ADN HP) ainsi qu'éventuellement les conservateurs et des parfums.

L'invention permet d'obtenir des liposomes d'eaux thermales parfaitement stabilisés dans un gel.

Ces formulations obtenues selon la présente invention, peuvent être utilisées en dermocosmétologie notamment par application topique lorsque la composition comporte un véhicule pour application topique, il peut s'agir de gel conditionné en tube ou système mécanique à pompe, ou de composition aérosol délivrée sous forme de gel.

Les exemples suivants sont destinés à illustrer l'invention sans pour autant présenter un caractère limitatif.

**EXEMPLE 1 : Préparation du lot de 5 kg de liposomes d'eau d'AVENE à 2 % de lipides.**

**Matière première :**

1 - Phase organique
- Phospholipide (Lipoïde 80)o SEPPIC          100 g
- Cholestérol BP          15 g
- Ethanol 95°          2,5 litres

2 - Phase aqueuse
- Eau d'AVENE          5 litres
- EDTA disodique          10 g

**Mode opératoire :**

1 - Préparation de la phase organique I

Dans 2,5 litres d'éthanol, on introduit sous forte agitation à température ambiante, 100 g de phospholipide, 10 g de cholestérol et 10 g de phénonim. On poursuit l'agitation 1/2 heure jusqu'à dissolution et obtention d'une phase homogène, jaune pâle.

2 - Préparation de la phase aqueuse II

Dans 5 litres d'eau d'Avène, on introduit sous agitation, 10 g d'EDTA disodique.

3 - Préparation des liposomes

On introduit la phase organique I à l'aide d'un Kremlin et d'une pompe péristaltique, par filets, dans la phase aqueuse II.

L'addition sous forte agitation à l'aide d'un Rayneri dure 15 mn. La phase organique doit être introduite en dehors dlu cône d'agitation. Il se forme une phase laiteuse.

On évapore sous pression réduite 2,7 litres (éthanol + eau). La température du bain-marie est de 50°C. On obtient une solution laiteuse de 4,8 litres à 2 % de lipides que l'on complète à 5 litres avec de l'eau d'AVENE.

**EXEMPLE 2 : Préparation de la composition de liposomes d'eau thermale stabilisés par un gel d'ADN HP.**

A la solution laiteuse précédente contenant 2 % de lipides, on introduit sous agitation modérée et par petites fractions, 100 g d'ADN Hp (commercialisé par la société JAVERNECH). La dissolution s'effectue lentement à température ambiante. Après 1 heure d'agitation, on obtient une formulation stabilisée contenant 2 % de gel d'ADN Hp et 2 % de liposomes.

**EXEMPLE 3 : Formulations**

Dans les formulations ci-après, la quantité (en poids) d'eau thermale encapsulée dans les liposomes est de l'ordre de 3 fois celle des lipides constituant les liposomes.

FORMULATION 1 :

| | | |
|---|---|---|
| Lipides | | 2 % |
| ADN HP | | 2 % |
| Phénonip | | 0,5 % |
| EDTA | | 0,2 % |
| Eau d'Avène | q.s.p. | 100 |

FORMULATION 2 :

| | | |
|---|---|---|
| Lipides | | 2 % |
| ADN HP | | 0,5 % |
| Phenonip | | 0,5 % |
| EDTA | | 0,2 % |
| Eau d'Avène | q.s.p. | 100 |

FORMULATION 3 :

| | | |
|---|---|---|
| Lipides | | 2 % |
| ADN HP | | 0,1 % |
| Phenonip | | 0,5 % |
| EDTA | | 0,2 % |
| Eau d'Avène | q.s.p. | 100 |

FORMULATION 4 :

| | |
|---|---|
| Lipides | 0,5 % |
| ADN HP | 0,2 % |

| Parahydroxybenzoate de butyle | | 0,2 % |
| Eau florale | | 1 % |
| Eau d'Avène | q.s.p. | 100 |

**FORMULATION 5 :**

| Lipides | | 2 % |
| ADN HP | | 0,5 % |
| Acide sorbique | | 0,3 % |
| Eau de Cauterets | q.s.p. | 100 |

**FORMULATION 6 :**

| Lipides | | 1 % |
| ADN HP | | 0,5 % |
| Vitamine C | | 1 % |
| Phenonip | | 0,5 % |
| Eau florale | | 1 % |
| Eau de Cauterets | q.s.p. | 100 |

**FORMULATION 7 :**

| Lipides | | 2 % |
| ADN HP | | 0,5 % |
| Extrait de Ginkgo | | 1 % |
| Phenonip | | 0,5 % |
| Eau florale | | 1 % |
| Eau thermale | q.s.p. | 100 |

**FORMULATION 8 :**

| Lipides | | 0,1 % |
| ADN HP | | 0,5 % |
| Huile de bourrache | | 1 % |
| Phenonip | | 1 % |
| Eau thermale | q.s.p. | 100 |

**FORMULATION 9 :**

| Lipides | | 10 % |
| ADN HP | | 5 % |
| Vitamine E | | 0,5 % |
| Phenonip | | 1 % |
| Eau thermale | q.s.p. | 100 |

**EXEMPLE 4 : Etude de stabilité**

L'étude de stabilité a été effectuée à 40°C sur des formulations contenant 2 % de liposomes d'eau thermale d'Avène.

La visualisation est réalisée au microscope électronique, cette visualisation est effectuée chaque mois après avoir procédé à la coloration négative des liposomes à l'aide d'une solution à 2 % de phosphotungstate de sodium.

Les résultats de cette étude sont résumés dans le tableau suivant :

7

| temps/mois<br>gélifiant | 1 | 2 | 3 | 4 | 5 | 6 | 7 | 8 | 9 | 10 | 11 | 12 |
|---|---|---|---|---|---|---|---|---|---|---|---|---|
| Gélatine 5 % | + | ± | - | | | | | | | | | |
| Carbopol 941 (0,5%) | + | + | ± | - | | | | | | | | |
| Carbopol 910 (0,5%) | + | + | ± | - | | | | | | | | |
| Eudispert HV (0,5%) | + | + | + | ± | - | | | | | | | |
| Eudispert MV (0,5%) | + | + | ± | - | | | | | | | | |
| Dimethycone copolyol (2 %) | + | + | + | ± | - | | | | | | | |
| Collagène (5 %) | + | + | + | ± | - | | | | | | | |
| ADN HP (0,1 %) | + | + | + | + | + | + | + | + | ± | - | | |
| ADN HP (0,5 %) | + | + | + | + | + | + | + | + | + | + | + | + |
| ADN HP (2 %) | + | + | + | + | + | + | + | + | + | + | + | + |

+ = Bonne stabilité (liposome 300 + 30 nm)

± = Augmentation taille des liposomes (par fusion membranaire)

- = Séparation des phases (instabilité de la formule).

Les observations photographiques montrent que les liposomes sont bien dispersés dans un réseau de l'ADN HP sans que leur forme et leur taille soient altérées et confirme la stabilité surprenante des liposomes dans cette formulation.

Des autres expériences réalisées, on arrive au résultat que la présence d'ADN selon l'invention permet d'obtenir une stabilisation remarquable des liposomes d'eau minérale selon l'invention lors de leur préparation ainsi que dans les formulations galéniques.

Un taux de 0,5 à 2% d'ADN H.P. permet de stabiliser les liposomes pendant 24 mois, alors qu'avec les gélifiants clasiques du type Carbopol et/ou Collagène, la stabilité des membranes liposomiales est maintenue au maximum 6 mois.

Cette stabilité peut être expliquée par la réticulation des fibres d'ADN H.P. en milieu aqueux qui permet la dispersion des liposomes et empêche la fusion des vésicules lipidiques.

## EXEMPLE 5 : Activité pharmaceutique et chimique

La vectorisation de l'eau minérale sous forme liposomiale selon l'invention permet d'une façon surprenante de potentialiser l'activité pharmacologique et clinique de cette eau.

Les résultats obtenus sont résumés ci-après.

### 1. Inhibition de la dégranulation des basophiles humains.

La dégranulation des basophiles humains est observée selon le protocole suivant :

Obtention de basophiles sensibilisés à un antigène donné à partir de prélèvements de malades allergiques ou après sensibilisation pasive de basophiles de donneurs par un sérum riche en IgE spécifiques.

Lorsque les basophiles sensibilisés proviennent de malades allergiques, il est nécessaire de procéder à un enrichissement par simple sédimentation à 1 g et centrifugation du plasma riche en leucocytes. Le culot leucocytaire contient de 1500 à 3000 basophiles/mm$^3$.

Les leucocytes obtenus sont alors mis en suspension dans un tampon minéral puis centrifugés.

L'antigène sensibilisant est alors dilué dans le RPMI 1640 (flows Labo) (7 dilutions de 5 en 5) à partir d'une concentration par exemple de 10-3 s'il s'agit d'extraits glycérinés.

Afin d'étudier la vectorisation de l'eau d'Avène sur ce basophile-allergène sensibilisant, l'eau pure et de l'eau distillée sont mises en contact avec un aliquot du culot cellulaire et incubées 30 mn à 25°C. Après ce temps d'incubation, la suspension cellulaire est mélangée volume à volume avec les dilutions de l'antigène, plus un contrôle sans l'antigène.

Le mélange cellules-antigène est mis à incuber 15 mn à 37°C puis coloré par le bleu de toluidine.

Les basophiles non dégranulés sont alors dénombrés dans des hémocytonitres de Malassez ou de Fuchs-Rosenthal.

### Résultats

Sur le tableau suivant figurent les résultats exprimés en pourcentage maximum de dégranulation pour 15 tests de dégranulation en présence de divers pneumallergènes. Le pourcentage de dégranulation moyen pour les 15 expériences est de 57,1% pour les témoins eau distillée et eau d'Avène de 29,3% et de 14,1% pour les tests réalisés avec des liposomes stabilisés. (Cette différence est hautement significative p < 0,01).

| Eau distillée | Eau d'Avène | Eau d'Avène liposomes ADN H.P. (5%) |
|---------------|-------------|-------------------------------------|
| 57,1% | 29,3% | 14,1% |

### Conclusion

La vectorisation de l'eau d'Avène par les liposomes permet une potentialisation de 100% de l'effet inhibiteur de la dégranulation des basophiles.

### 2. Pharmacoclinique

Une potentialisation de l'activité anti-irritante chez l'homme a été étudiée à partir d'un modèle d'irritation cutanée provoquée par le Lauryl Sulfate de Sodium (LSS).

Produits testés :        (a) Eau distillée
(b) Eau d'Avène
(c) Eau d'Avène (liposomes ADN H.P. - 5%)

Les trois produits à étudier sont utilisés comme solvant du LSS afin de réaliser des solutions de même concentration que la solution témoin de LSS constituant le modèle d'irritation. Ils sont appliqués ainsi que le témoin sous patch-test occlusif, pendant 24 heures.

L'évaluation de l'intensité de l'irritation cutanée est réalisée par mesure des variations du flux sanguin cutané en Vélocimétrie Laser Doppler (VLD).

<u>Protocole</u> (nombre de sujets : 20)

Trois solutions de LSS à 0,75% sont préparées à l'aide des produits à étudier (a-b-c). La solution témoin de LSS et les solutions de a, b et c sont appliquées de manière randomisée à raison de 65 $\mu$l/cm$^2$ sur un disque de papier filtre.

Un pansement occlusif est ensuite maintenu pendant 24 heures. Après retrait des pansements, la peau est laissée à l'air libre pendant 30 mn avant le début des mesures afin d'éliminer tout effet éventuel de l'occlusion.

La mesure du flux sanguin cutané est réalisée par enregistrement VLD durant 10 mn sur chaque zone, d'une part avant l'application des patches pour obtenir la ligne de base physiologique et, d'autre part, 30 mn après le retrait des patches.

Les résultats sont exprimés par la moyenne des aires sous la courbe.

Le pourcentage d'inhibition de l'inflammation par le LSS est calculé à l'aide de la formule suivante :

$$\% = \frac{\text{ASC LSS} - \text{ASC p.}}{\text{ASC LSS}} \, 100$$

ASC LSS = Aire sous la courbe pour la solution de LSS seule

ASC p = Aire sous la courbe pour les solutions des produits a, b et c

Afin de déterminer si l'activité des produits étudiés est significative, une étude statistique au moyen du test t apparié de Student est effectuée sur les résultats obtenus.

<u>Résultats</u>

Pourcentage d'inhibition de l'irritation induite par LSS

| a | b | c |
|---|---|---|
| 14 NS | 39,8 (S) | 82,2 (S) |

L'étude clinique confirme la potentialisation très nette (> 100%) de l'activité anti-inflammatoire de l'eau d'Avène vectorisée par des liposomes.

## Revendications

**Revendications pour les Etats contractants suivants : AT, BE, CH, DE, DK, FR, GB, IT, LI, LU, NL, SE**

**1.** Composition à base d'eau thermale caractérisée en ce qu'elle comporte des liposomes d'eau thermale stabilisés dans un gel d'acide desoxyribonucléique.

**2.** Composition selon la revendication 1 caractérisée en ce que l'acide desoxyribonucléique utilisé est hautement polymérisé (ADN HP).

**3.** Composition selon l'une des revendications 1 à 2, caractérisée en ce qu'elle contient 0,1 % à 10 % de lipides entrant dans la composition des liposomes.

**4.** Composition selon l'une des revendications 1 à 3, caractérisée en ce qu'elle contient de 0,1 à 10 % d'ADN.

**5.** Composition selon l'une des revendications 1 à 4, caractérisée en ce qu'elle contient un principe actif associé qui est un agent antibactérien et plus particulièrement le phenonip, l'EDTA, le parahydroxybenzoate de butyle, l'acide sorbique.

**6.** Composition selon l'une des revendications 1 à 5, caractérisée en ce qu'elle contient un principe actif associé qui est la vitamine E, la vitamine C, l'huile de bourrache, l'huile d'argan, un extrait de ginkgo biloba.

**7.** Composition selon les revendications 1 à 6, caractérisée en ce que l'eau thermale utilisée est plus particulièrement l'eau de Cauterets ou de l'eau d'Avène.

**8.** Compostion selon l'une des revendications 5 à 7, caractérisée en ce que la compostion comporte 0,1 à 3 % en poids dudit principe actif associé.

**9.** Composition selon les revendications 1 à 8 caractérisée en ce qu'elle comporte en outre un ou des véhicules appropriés pour une utilisation en cosmétologie notamment en dermocosmétologie.

**10.** Procédé de préparation d'une composition comportant un gel de liposomes selon l'une des revendications précédentes caractérisée en ce qu'il comporte les étapes suivantes :
1) On prépare une phase liquide constituée essentiellement par une solution de lipides amphiphiles et éventuellement un dit principe actif associé de nature lipophile dans un solvant organique et plus particulièrement l'éthanol,
2) Cette phase est alors ajoutée sous agitation modérée, dans une solution d'eau thermale contenant éventuellement un dit principe actif associé de nature hydrophile,
3) Après évaporation sous pression réduite, on obtient une suspension de concentration voulue en liposomes,
4) A la suspension précédente, on introduit sous agitation modérée dudit ADN.

**Revendications pour les Etats contractants suivants : ES, GR**

**1.** Procédé de préparation d'une composition à base d'eau thermale qui comporte des liposomes d'eau thermale stabilisés dans un gel d'acide désoxyribonucléique (ADN), caractérisé en ce que l'on prépare une suspension de liposomes d'eau thermale et l'on y introduit ledit ADN.

**2.** Procédé selon la revendication 1 caractérisé en ce que l'acide désoxyribonucléique utilisé est hautement polymérisé (ADN HP).

**3.** Procédé selon l'une des revendications 1 à 2, caractérisé en ce que 0,1% à 10% de lipides entrent dans la composition des liposomes.

**4.** Procédé selon l'une des revendications 1 à 3, caractérisé en ce que la composition contient de 0,1 à 10% d'ADN.

**5.** Procédé selon l'une des revendications 1 à 4, caractérisé en ce qu'un principe actif est ajouté, qui est un agent antibactérien et, plus particulièrement, le phénonip, l'EDTA, le parahydroxybenzoate de butyle, l'acide sorbique.

**6.** Procédé selon l'une des revendications 1 à 5, caractérisé en ce qu'un principe actif est ajouté, qui est la vitamine E, la vitamine C, l'huile de bourrache, l'huile d'argan, un extrait de ginkgo biloba.

**7.** Procédé selon les revendications 1 à 6, caractérisé en ce que l'eau thermale utilisée est plus particulièrement l'eau de Cauterets ou de l'eau d'Avène.

**8.** Procédé selon l'une des revendications S à 7, caractérisé en ce qu'on ajoute de 0,1 à 3% en poids dudit principe actif associé.

**9.** Procédé selon les revendications 1 à 8 caractérisé en ce qu'on ajoute en outre un ou des véhicule(s) approprié(s) pour une utilisation en cosmétologie, notamment en dermocosmétologie.

**10.** Procédé de préparation d'une composition comportant un gel de liposomes selon l'une des revendications précédentes, caractérisé en ce qu'il comporte les étapes suivantes:
1) On prépare une phase liquide constituée essentiellement par une solution de lipides amphiphiles et éventuellement un dit principe actif associé de nature lipophile dans un solvant organique et, plus particulièrement, l'éthanol,
2) Cette phase est alors ajoutée sous agitation modérée, dans une solution d'eau thermale contenant éventuellement un dit principe actif associé de nature hydrophile,

3) Après évaporation sous pression réduite, on obtient une suspension de concentration voulue en liposomes,

4) A la suspension précédente, on introduit sous agitation modérée dudit ADN.

## Patentansprüche

**Patentansprüche für folgende Vertragsstaaten : AT, BE, CH, DE, DK, FR, GB, IT, LI, LU, NL, SE**

1. Zusammensetzung auf der Grundlage von Heilwasser, dadurch gekennzeichnet, daß es in einem Desoxyribonucleinsäure-Gel stabilisierte Heilwässerliposome umfaßt.

2. Zusammensetzung nach Anspruch 1, dadurch gekennzeichnet, daß die verwendete Desoxyribonucleinsäure stark polymerisiert (DNA HP) ist.

3. Zusammensetzung nach einem der Ansprüche 1 bis 2, dadurch gekennzeichnet, daß sie 0,1 % bis 10 % Lipide, die in der Liposomzusammensetzung vorliegen, enthält.

4. Zusammensetzung nach einem der Ansprüche 1 bis 3, dadurch gekennzeichnet, daß sie 0,1 bis 10 % DNA enthält.

5. Zusammensetzung nach einem der Ansprüche 1 bis 4, dadurch gekennzeichnet, daß sie eine assoziierte Aktivsubstanz enthält, die ein antibakterielles Mittel ist und insbesondere Phenonip, EDTA, Butyl-para-hydroxybenzoat, Sorbinsäure.

6. Zusammensetzung nach einem der Ansprüche 1 bis 5, dadurch gekennzeichnet, daß eine assoziierte Aktivsubstanz, die Vitamin E, Vitamin C, Gurkenkrautöl, Arganöl, ein Extrakt von Ginkgo Biloba ist.

7. Zusammensetzung nach einem der Ansprüche 1 bis 6, dadurch gekennzeichnet, daß das verwendete Thermalwasser insbesondere Wasser aus Cauterets oder Wasser aus Avène.

8. Zusammensetzung nach einem der Ansprüche 5 bis 7, dadurch gekennzeichnet, daß die Zusammensetzung 0,1 bis 3 Gew.-% der assoziierten Aktivsubstanz umfaßt.

9. Zusammensetzung nach einem der Ansprüche 1 bis 8, dadurch gekennzeichnet, daß sie einen oder mehrere für eine Verwendung in der Kosmetik, insbesondere in der Dermatologie, geeignete Vehikel umfaßt.

10. Verfahren zur Herstellung einer Zusammensetzung, umfassend ein Liposomgel nach einem der vorangegangenen Ansprüche, dadurch gekennzeichnet, daß es die folgenden Schritte umfaßt:

    1) man stellt eine flüssige Phase, die im wesentlichen aus einer Lösung von amphiphilen Lipiden und gegebenenfalls eines der genannten assoziierten Aktivsubstanzen lipophiler Natur besteht, in einem organischen Lösungsmittel und insbesondere Ethanol, her,

    2) diese Phase wird dann unter mäßigem Rühren in eine Lösung von Heilwässern, die gegebenenfalls eines der assoziierten hydrophilen Aktivsubstanzen enthält, hinzugefügt,

    3) nach dem Abdampfen unter reduziertem Druck erhält man eine Suspension mit gewünschter Konzentration an Liposomen,

    4) zu der voranstehenden Suspension fügt man unter moderatem Rühren die DNA.

**Patentansprüche für folgende Vertragsstaaten : ES, GR**

1. Verfahren zur Herstellung einer Zusammensetzung auf der Grundlage von Heilwasser, die in einem Desoxyribonucleinsäure-Gel stabilisierte Heilwässerliposome umfaßt, dadurch gekennzeichnet, daß man eine Suspension aus Heilwässerliposomen herstellt und die DNA hineingibt.

2. Verfahren nach Anspruch 1, dadurch gekennzeichnet, daß die verwendete Desoxyribonucleinsäure stark polymerisiert (DNA HP) ist.

3. Verfahren nach einem der Ansprüche 1 bis 2, dadurch gekennzeichnet, daß 0,1 % bis 10 % Lipide in der Liposomzusammensetzung vorliegen.

4. Verfahren nach einem der Ansprüche 1 bis 3, dadurch gekennzeichnet, daß die Zusammensetzung 0,1 bis 10 % DNA enthält.

5. Verfahren nach einem der Ansprüche 1 bis 4, dadurch gekennzeichnet, daß eine assoziierte Aktivsubstanz hinzugefügt wird, die ein antibakterielles Mittel ist und insbesondere Phenonip, EDTA, Butyl-para-hydroxybenzoat, Sorbinsäure.

6. Verfahren nach einem der Ansprüche 1 bis 5, dadurch gekennzeichnet, daß eine assoziierte Aktivsubstanz hinzugefügt wird, die Vitamin E, Vitamin C, Gurkenkrautöl, Arganöl, ein Extrakt von Ginkgo Biloba ist.

7. Verfahren nach einem der Ansprüche 1 bis 6, dadurch gekennzeichnet, daß das verwendete Thermalwasser insbesondere Wasser aus Cauterets oder Wasser aus Avène.

8. Verfahren nach einem der Ansprüche 5 bis 7, dadurch gekennzeichnet, daß man 0,1 bis 3 Gew.-% der assoziierten Aktivsubstanz hinzufügt.

9. Verfahren nach einem der Ansprüche 1 bis 8, dadurch gekennzeichnet, daß man einen oder mehrere für eine Verwendung in der Kosmetik, insbesondere in der Dermatologie, geeignete Vehikel hinzufügt.

10. Verfahren zur Herstellung einer Zusammensetzung, umfassend ein Liposomgel nach einem der vorangegangenen Ansprüche, dadurch gekennzeichnet, daß es die folgenden Schritte umfaßt:
1) man stellt eine flüssige Phase, die im wesentlichen aus einer Lösung von amphiphilen Lipiden und gegebenenfalls eines der genannten assoziierten Aktivsubstanzen lipophiler Natur besteht, in einem organischen Lösungsmittel und insbesondere Ethanol, her,
2) diese Phase wird dann unter mäßigem Rühren in eine Lösung von Heilwässern, die gegebenenfalls eines der assoziierten hydrophilen Aktivsubstanzen enthält, hinzugefügt,
3) nach dem Abdampfen unter reduziertem Druck erhält man eine Suspension mit gewünschter Konzentration an Liposomen,
4) zu der voranstehenden Suspension fügt man unter moderatem Rühren die DNA.

**Claims**

**Claims for the following Contracting States : AT, BE, CH, DE, DK, FR, GB, IT, LI, LU, NL, SE**

1. Composition based on thermal water characterized in that it contains liposomes of thermal water stabilized in a deoxyribonucleic acid gel.

2. Composition according to Claim 1, characterized in that the deoxyribonucleic acid used is highly polymerized (HP DNA).

3. Composition according to one of Claims 1 and 2, characterized in that it contains 0.1% to 10% lipids which enter into the composition of the liposomes.

4. Composition according to one of Claims 1 to 3, characterized in that it contains 0.1 to 10% DNA.

5. Composition according to one of Claims 1 to 4, characterized in that it contains an associated active ingredient which is an antibacterial agent and more particularly phenonip, EDTA, butyl para-hydroxybenzoate, sorbic acid.

6. Composition according to one of Claims 1 to 5, characterized in that it contains an associated active ingredient which is vitamin E, vitamin C, borage oil, argan oil, a ginkgo biloba extract.

7. Composition according to Claims 1 to 6, characterized in that the thermal water used is more particularly Cauterets or Avene water.

8. Composition according to one of Claims 5 to 7, characterized in that the composition contains 0.1 to 3% by weight of the said associated active ingredient.

9. Composition according to Claims 1 to 8, characterized in that it contains, in addition, one or more vehicles appropriate for use in cosmetology especially in dermocosmetology.

10. Process for the preparation of a composition containing a liposome gel according to one of the preceding claims, characterized in that it comprises the following steps:
1) A lipid phase consisting essentially of a solution of amphiphilic lipids and optionally a said associated active ingredient of lipophilic nature is prepared in an organic solvent and more particularly ethanol,
2) this phase is then added, with gentle stirring, to a solution of thermal water optionally containing a said associated active ingredient of hydrophilic nature,
3) after evaporation under reduced pressure, a suspension of desired liposome concentration is obtained,
4) the said DNA is introduced into the above suspension with gentle stirring.

**Claims for the following Contracting States : ES, GR**

1. Process for the preparation of a composition based on thermal water which contains liposomes of thermal water stabilized in a deoxyribonucleic acid (DNA) gel, characterized in that a suspension of liposomes of thermal water is prepared and the said DNA is introduced therein.

2. Process according to Claim 1, characterized in that the deoxyribonucleic acid used is highly polymerized (HP DNA).

3. Process according to one of Claims 1 and 2, characterized in that 0.1% to 10% lipids enter into the composition of the liposomes.

4. Process according to one of Claims 1 to 3, characterized in that the composition contains 0.1 to 10% DNA.

5. Process according to one of Claims 1 to 4, characterized in that an active ingredient is added, which is an antibacterial agent and more particularly phenonip, EDTA, butyl para-hydroxybenzoate, sorbic acid.

6. Process according to one of Claims 1 to 5, characterized in that an active ingredient is added, which is vitamin E, vitamin C, borage oil, argan oil, a ginkgo biloba extract.

7. Process according to Claims 1 to 6, characterized in that the thermal water used is more particularly Cauterets or Avene water.

8. Process according to one of Claims 5 to 7, characterized in that 0.1 to 3% by weight of the said associated active ingredient is added.

9. Process according to Claims 1 to 8, characterized in that, in addition, one or more vehicles appropriate for use in cosmetology, especially in dermocosmetology, is added.

10. Process for the preparation of a composition containing a liposome gel according to one of the preceding claims, characterized in that it comprises the following steps:
1) A lipid phase consisting essentially of a solution of amphiphilic lipids and optionally a said associated active ingredient of lipophilic nature is prepared in an organic solvent and more particularly ethanol,
2) this phase is then added, with gentle stirring, to a solution of thermal water optionally containing a said associated active ingredient of hydrophilic nature,
3) after evaporation under reduced pressure, a suspension of desired liposome concentration is obtained,
4) the said DNA is introduced into the above suspension with gentle stirring.